(19) Europäisches Patentamt
European Patent Office
Office européen des brevets

(11) EP 2 362 477 A1

(12) **EUROPEAN PATENT APPLICATION**

(43) Date of publication:
**31.08.2011 Bulletin 2011/35**

(51) Int Cl.:
***H01M 10/052*** *(2010.01)* ***H01M 10/0567*** *(2010.01)*

(21) Application number: **11154818.6**

(22) Date of filing: **17.02.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **18.02.2010 JP 2010033609**

(71) Applicant: **Sanyo Electric Co., Ltd.**
**Osaka 570-8677 (JP)**

(72) Inventors:
- **Tuduki, Kouhei**
  **Moriguchi-shi Osaka 5708677 (JP)**
- **Yamamoto, Hidekazu**
  **Moriguchi-shi Osaka 5708677 (JP)**
- **Sunano, Taizou**
  **Moriguchi-shi Osaka 5708677 (JP)**
- **Kamino, Maruo**
  **Moriguchi-shi Osaka 5708677 (JP)**

(74) Representative: **Glawe, Delfs, Moll**
**Patent- und Rechtsanwälte**
**Postfach 26 01 62**
**80058 München (DE)**

(54) **Non-aqueous electrolyte secondary battery**

(57) A non-aqueous electrolyte secondary battery includes a positive electrode (1), a negative electrode (2) containing a negative electrode active material, a separator 3 interposed between the electrodes (1) and (2), and a non-aqueous electrolyte containing a non-aqueous solvent and a solute dissolved in the solvent. The non-aqueous electrolyte contains a compound represented by the following chemical formula (1):

Chemical formula (1)

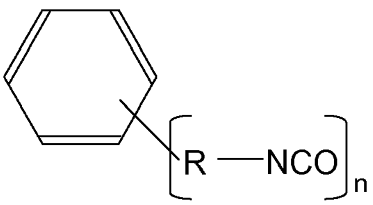

,

wherein n is an integer of from 2 to 6, each R represents a linear saturated hydrocarbon that may be an unsubstituted or may have a substituted group, and the Rs may be the same or different groups.

EP 2 362 477 A1

Printed by Jouve, 75001 PARIS (FR)

**Description**

BACKGROUND OF THE INVENTION

Field of the Invention

The present invention relates to non-aqueous electrolyte secondary batteries, and more particularly to a non-aqueous electrolyte used for the batteries.

Description of Related Art

Mobile information terminal devices such as mobile telephones, notebook computers, and PDAs have become smaller and lighter at a rapid pace in recent years. This has led to a demand for higher capacity secondary batteries as the drive power source for the mobile information terminal devices. Non-aqueous electrolyte secondary batteries, which have high energy density among secondary batteries, have achieved higher capacity year by year. However, the power consumption of the mobile information terminals has been increasing, as they tend to have increasing numbers of features and functions. Accordingly, there is a strong demand for a non-aqueous electrolyte secondary battery with higher capacity and higher performance such that it can enable the devices to operate for longer hours at high output power.

Conventionally, the research and development efforts to improve the capacity of the non-aqueous electrolyte secondary batteries have centered around thickness reduction of the components that do not relate to the power-generating element, such as battery can, separator, and current collector (aluminum foil or copper foil), as well as increasing of the filling density of active material (improvements in electrode filling density). These techniques, however, seem to be approaching their limits, and fundamental improvements such as finding alternative materials have become necessary to achieve higher capacity. A known example of the attempts to provide a solution to such problems is a battery that uses a carbon material such as graphite as the negative electrode material. A battery that uses a silicon alloy and other alloy-based materials as the negative electrode material has also been proposed to obtain a higher capacity.

However, when the negative electrode material contains graphite, silicon, or the like and the non-aqueous electrolyte contains a solvent of carbonate or the like, the solvent of carbonate or the like undergoes a reductive decomposition on the negative electrode surface if the battery is stored at high temperature for a long time. As a consequence, these batteries have poor high-temperature storage performance.

In view of such circumstances, Japanese Published Unexamined Patent Application No. 2007-242411 proposes a battery that uses an electrolyte containing a diisocyanate compound having an aliphatic carbon chain.

However, even with the battery described in Japanese Published Unexamined Patent Application No. 2007-242411, which uses an electrolyte containing a diisocyanate compound, the high-temperature storage performance cannot be improved sufficiently.

BRIEF SUMMARY OF THE INVENTION

Accordingly, it is an object of the present invention to provide a non-aqueous electrolyte secondary battery that can inhibit the reductive decomposition reaction on the negative electrode even when the battery is stored at high temperature for a long time and thereby improve high-temperature storage performance significantly.

In order to accomplish the foregoing and other objects, the present invention provides a non-aqueous electrolyte secondary battery comprising: a positive electrode; a negative electrode containing a negative electrode active material; a separator interposed between the positive and negative electrodes; and a non-aqueous electrolyte containing a non-aqueous solvent and a solute dissolved in the solvent, wherein the non-aqueous electrolyte contains a compound represented by the following chemical formula (1):

Chemical formula (1)

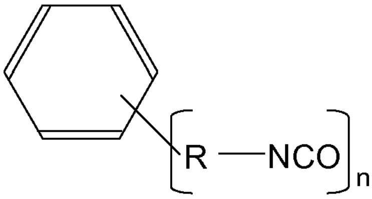

,

wherein n is an integer of from 2 to 6, each R represents a linear saturated hydrocarbon that may be an unsubstituted group or may have a substituted group, and the Rs may be the same or different groups.

The present invention makes it possible to inhibit the reductive decomposition reaction on the negative electrode even when the battery is stored at high temperature for a long time and to thereby improve the high-temperature storage performance significantly.

BRIEF DESCRIPTION OF THE DRAWINGS

Fig. 1 is a front view illustrating a non-aqueous electrolyte secondary battery according to the present invention; and
Fig. 2 is a cross-sectional view taken along line A-A in Fig. 1.

DETAILED DESCRIPTION OF THE INVENTION

A non-aqueous electrolyte secondary battery according to the invention comprises: a positive electrode; a negative electrode containing a negative electrode active material; a separator interposed between the positive and negative electrodes; and a non-aqueous electrolyte containing a non-aqueous solvent and a solute dissolved in the solvent. The non-aqueous electrolyte contains a compound represented by the following chemical formula (1):

Chemical Formula (1)

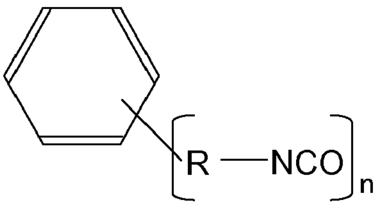

,

In the formula, n is an integer of from 2 to 6, each R represents a linear saturated hydrocarbon that may be an unsubstituted group or may have a substituted group, and the Rs may be the same or different groups.

The just-described compound (isocyanate compound) has the following features.

(1) It has a plurality of isocyanate structures.
(2) It is an aromatic hydrocarbon derivative.
(3) The isocyanato groups are bonded to an aromatic hydrocarbon via a linear saturated hydrocarbon.

When the compound with the just-described features is contained in the non-aqueous electrolyte, a desirable surface film can be formed on the electrode surface. Thereby, the reliability of the battery stored at high temperature for a long time can be improved dramatically. It is believed that the reason is as follows.

It is known that a compound having an isocyanato group bonds with the hydroxy groups existing on the surface of the negative electrode comprising carbon or silicon, forming a surface film on the surface of the negative electrode. However, when the compound has only one isocyanate structure, it has only one bonding site to the hydroxy group

existing on the surface of the negative electrode, so the cohesive strength of the compound with the negative electrode is weak. On the other hand, when the compound has a plurality of isocyanate structures, it has a plurality of bonding sites to the hydroxy group existing on the surface of the negative electrode, so the cohesive strength of the compound with the negative electrode is strong. In addition, when the amount of the addition agent is increased, the battery performance tends to be affected adversely. For this reason, in order to form a desirable surface film with a small amount of the addition agent, it is necessary that the compound have a plurality of isocyanate structures, as mentioned in (1) above.

In addition, when the compound having isocyanato groups is a chain or cyclic aliphatic hydrocarbon derivative, it has no advantageous effect on the storage performance. The reason is that even when a surface film on the negative electrode surface is formed by the chain or cyclic aliphatic hydrocarbon derivative, the surface film cannot cover the negative electrode surface sufficiently because the chain or cyclic aliphatic hydrocarbon derivative has a strained structure. Consequently, the surface film cannot prevent excessive reactions on the negative electrode surface with the electrolyte. In contrast, in relation to the feature of having a plurality of isocyanate structures as described in (1) above, the aromatic hydrocarbon derivative has a planar structure of the benzene ring, so the aromatic hydrocarbon is arranged along the negative electrode surface. Therefore, the resulting surface film can cover the negative electrode surface sufficiently. As a result, it becomes possible to inhibit the reductive decomposition reaction on the negative electrode surface.

For these reasons, it is necessary that the additive compound be an aromatic hydrocarbon derivative, as described in (2) above.

Moreover, when the isocyanato groups are bonded to the aromatic hydrocarbon via the linear saturated hydrocarbon, the relative position of the isocyanato groups and the aromatic hydrocarbon can vary since the linear saturated hydrocarbon can freely rotate around the bond. As a result, it becomes possible to increase the degree of spatial freedom of the isocyanato groups, which are the reactive sites. On the other hand, when the isocyanato groups are bonded directly to the aromatic hydrocarbon, not via the linear saturated hydrocarbon, the relative position of the isocyanato groups and the aromatic hydrocarbon cannot vary. As a consequence, it is impossible to increase the degree of spatial freedom of the isocyanato groups, which are the reaction sites.

Thus, as described in (3) above, it is necessary that in the additive compound, the isocyanato groups are bonded to the aromatic hydrocarbon via the linear saturated hydrocarbon.

It is desirable that the negative electrode contain silicon.

The negative electrode active material, silicon, undergoes a large volumetric change during charge and discharge. For this reason, when a conventional surface film is used, the surface film is destroyed because of the expansion of the negative electrode active material during charge. However, the diisocyanate compound having a structure represented by the foregoing formula can alleviate the stress applied to the compound even when the negative electrode active material undergoes a volumetric change, because it has a linear saturated hydrocarbon that is rotatable. As a result, it is made possible to inhibit the surface film from being destroyed even when the negative electrode active material contains silicon.

When silicon is used for the negative electrode active material, it is preferable that the negative electrode be manufactured by forming a silicon film on a negative electrode current collector by vacuum evaporation, or by sintering a negative electrode mixture layer that contains a binder and negative electrode active material particles containing silicon on the surface of a conductive metal foil current collector.

Examples of the negative electrode active materials that cause a large volumetric change during charge and discharge other than silicon include tin alloys and other alloy based materials, and when these materials are used as well, the present invention may be applied suitably.

It is desirable that the R in the chemical formula (1) be a linear saturated hydrocarbon having a carbon number of from 1 to 4. It is especially desirable that the R be a methylene group.

The greater the number of carbon atoms, the higher the degree of the spatial freedom and the higher the reactivity. However, when the number of carbon atoms is too great, the molecule becomes bulky, and the surface film formed by such molecules has an excessively high resistance. As a consequence, a surface film having desired performance cannot be formed. For this reason, it is desirable that the R be a linear saturated hydrocarbon having a carbon number of from 1 to 4, and it is especially desirable that the R be a methylene group.

It is desirable that n be 2 in the chemical formula (1). It is particularly desirable that the two R-NCOs in the chemical formula (1) be in the meta position.

It is desirable that the non-aqueous solvent contain a cyclic carbonate ester and a chain carbonate ester, and at least one of the carbonate esters contain fluorine.

In particular, when silicon is used for the negative electrode, it is desirable that at least one of the carbonates contain fluorine. Preferable examples of the cyclic carbonate ester containing fluorine include 4-fluoroethylene carbonate and 4,5-difluoroethylene carbonate. A preferable example of the chain carbonate ester is methyl 2,2,2-trifluoromethyl carbonate.

Examples of the solvent for the non-aqueous electrolyte include, but are not particularly limited to, cyclic carbonates such as ethylene carbonate, propylene carbonate, butylene carbonate, and vinylene carbonate; chain carbonates such as dimethyl carbonate, ethyl methyl carbonate, and diethyl carbonate; esters such as methyl acetate, ethyl acetate, propyl acetate, methyl propionate, ethyl propionate, and γ-butyrolactone; ethers such as 1,2-dimethoxyethane, 1,2-diethoxyethane, tetrahydrofuran, 1,2-dioxane, and 2-methyltetrahydrofuran; nitriles such as acetonitrile; and amides such as dimethylformamide. These solvents may be used either alone or in combination.

Other Embodiments

(1) In the present invention, examples of the solute of the non-aqueous electrolyte include, but are not particularly limited to: lithium compounds represented by the chemical formula $LiXF_y$ (wherein X is P, As, Sb, B, Bi, Al, Ga, or In; and y is 6 when X is P, As, or Sb; or y is 4 when X is B, Bi, Al, Ga, or In), such as $LiPF_6$, $LiBF_4$, and $LiAsF_6$; and lithium compounds such as $LiCF_3SO_3$, $LN(CF_3SO_2)_2$, $LN(C_2F_5SO_2)_2$, $LiN(CF_3SO_2)(C_4F_9SO_2)$, $LiC(CF_3SO_2)_3$, $LiC(C_2F_5SO_2)_3$, $LiCl0_4$, $Li_2B_{10}Cl_{10}$, and $Li_2B_{12}Cl_{12}$. Among them, $LiPF_6$ is particularly preferable.

(2) It is possible to use, as the positive electrode active material, lithium transition metal oxides such as $LiMn_2O_4$, $LiNiO_2$, and composite oxides thereof, other than $LiCoO_2$ and $LiMn_{1/3}Ni_{1/3}CO_{1/3}O_2$. These oxides may also be used alone or in combination.

When lithium cobalt oxide is used as the positive electrode active material, it is desirable that zirconium be added thereto. When lithium cobalt oxide is used, the crystal structure tends to become instable as the state of charge increases. Since an alloy negative electrode may be used in the present invention, the positive electrode potential tends to be higher than in the battery using a conventional graphite negative electrode when the charge voltage of the battery is the same, and the crystal structure of lithium cobalt oxide tends to degrade more easily. When zirconium is firmly adhered to the surface of the positive electrode, the cycle performance is made stable.

DESCRIPTION OF THE PREFERRED EMBODIMENTS

Hereinbelow, examples of the non-aqueous electrolyte secondary battery according to the present invention are described in detail. It should be construed, however, that the non-aqueous electrolyte secondary battery according to this invention is not limited to the following embodiments and examples but various changes and modifications may be made without departing from the scope of the invention.

*Preparation of Positive Electrode*

First, positive electrode active material powder in which Zr is firmly adhered to the surface of lithium-cobalt composite oxide ($LiCoO_2$) was prepared. The positive electrode active material powder, carbon material powder as a positive electrode conductive agent, and polyvinylidene fluoride as a positive electrode binder were weighed so that the mass ratio of the positive electrode active material, the positive electrode conductive agent, and the positive electrode binder became 95 : 2.5 : 2.5. Thereafter, they were added to N-methyl-2-pyrrolidone as a dispersion medium, and the mixture was kneaded, to prepare a positive electrode mixture slurry.

Next, the resultant positive electrode mixture slurry was applied onto both sides of a positive electrode current collector (thickness 15 μm, length 402 mm, width 50 mm) made of an aluminum foil. On the obverse side, the active material slurry was applied so that the length was 340 mm and the width was 50 mm. On the reverse side, the active material slurry was applied so that the length was 271 mm and the width was 50 mm. Next, the positive electrode mixture slurry was dried to form positive electrode mixture layers on the respective sides of the positive electrode current collector, and thereafter, the resultant material was calendered. The electrode thickness after the calendaring was 154 μm. The amount of the positive electrode mixture on the positive electrode current collector was 50 $mg/cm^2$, and the filling density of the positive electrode mixture was 3.6 g/cc. Lastly, a positive electrode current collector tab made of an aluminum flat plate (thickness 70 μm, a length 35 mm, and width 4 mm) was attached to a portion of the current collector on which the positive electrode mixture slurry was not applied, by thrust-and-press clamping. Thus, a positive electrode was prepared.

*Preparation of Negative Electrode*

First, silicon powder (average particle size 10 μm, purity 99.9%) as the source material for the active material, a conductive agent, polyimide as a binder, and N-methyl pyrrolidone were mixed together so that the mass ratio became 44.77 : 1.87 : 3.37 : 50, to prepare a negative electrode mixture slurry.

Next, the resultant negative electrode mixture slurry was applied onto the entire surfaces of a copper foil (thickness 20 μm, length 380 mm, width 52 mm, surface roughness Ra 1.0 μm) serving as a negative electrode current collector, and then dried. Subsequently, the resultant article was calendered and thereafter heat-treated under an argon atmosphere at 420°C for 10 hours, to thereby prepare a sintered negative electrode. The thickness of the sintered material (including the negative electrode current collector) was 56 μm. Therefore, it is believed that the thickness of the negative electrode active material layer (one side) was 18 μm [(56 μm - 20 μm)/2]. Lastly, a negative electrode current collector tab made of a nickel flat plate (thickness 70 μm, a length 35 mm, and width 4 mm) was attached to the negative electrode current collector by thrust-and-press clamping. Thus, a negative electrode was prepared.

*Preparation of Non-aqueous Electrolyte Solution*

$LiPF_6$ as an electrolyte salt was dissolved at a concentration of 1 mole/liter in a mixed solvent in which 4-fluoroethylene carbonate (FEC) and methyl ethyl carbonate (MEC) were mixed at a volume ratio of FEC : MEC = 20 : 80. Thereafter, an addition agent represented by the following chemical formula (2) was added at a concentration of 1 mass % with respect to the mass of the mixed solvent and dissolved therein, to thereby prepare a non-aqueous electrolyte.

Chemical Formula (2)

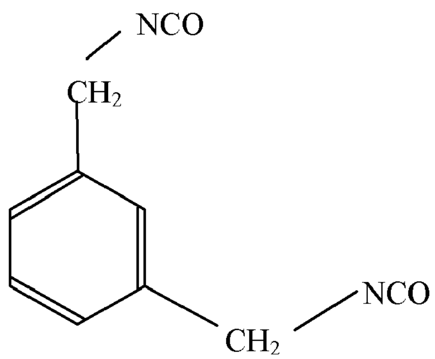

*Construction of Battery*

Using one sheet of the above-described positive electrode, one sheet of the above-described negative electrode, and two sheets of separators each made of porous polyethylene (thickness 22 μm, length 430 mm, width 54.5 mm), the positive electrode and the negative electrode were opposed to each other across the separators and bent at predetermined bending positions, to prepare a flat-type electrode assembly. At this time, the electrode assembly was coiled so that the positive and negative electrode current collector tabs were disposed at the outermost roll.

Next, the electrode assembly was placed in the space provided in aluminum laminate films serving as a battery case. Thereafter, the non-aqueous electrolyte was filled in the space, and thereafter, the aluminum laminate films were sealed by welding them together. Thus, a battery was prepared. The design capacity of the battery is 950 mAh when charged to 4.20 V.

The specific structure of the non-aqueous electrolyte secondary battery 11 is as follows. As illustrated in Figs. 1 and 2, a positive electrode 1 and a negative electrode 2 are disposed so as to oppose each other across separators 3. The non-aqueous electrolyte is impregnated in a flat-type electrode assembly comprising the positive electrode 1, the negative electrode 2, and the separator 3. The positive electrode 1 and the negative electrode 2 are connected to a positive electrode current collector tab 4 and a negative electrode current collector tab 5, respectively, so as to form a structure that enables charging and discharging as a secondary battery. The electrode assembly is disposed in a space within an aluminum laminate battery case 6 having a sealed part 7, at which opposing peripheral edges of the aluminum laminate films are heat sealed.

EXAMPLES

Example

A battery was fabricated in the same manner as described in the just-described embodiment.

The battery fabricated in this manner is hereinafter referred to as Battery A of the invention.

Comparative Example 1

A battery was fabricated in the same manner as described in Example above, except that the addition agent to be added to the non-aqueous electrolyte was an addition agent represented by the following chemical formula (3) (the amount of the addition agent was 1 mass % with respect to the mixed solvent).

The battery fabricated in this manner is hereinafter referred to as Comparative Battery Z1.

Chemical Formula (3)

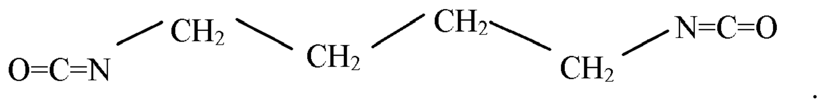

Comparative Example 2

A battery was fabricated in the same manner as described in Example above, except that the addition agent to be added to the non-aqueous electrolyte was an addition agent represented by the following chemical formula (4) (the amount of the addition agent was 1 mass % with respect to the mixed solvent).

The battery fabricated in this manner is hereinafter referred to as Comparative Battery Z2.

Chemical Formula (4)

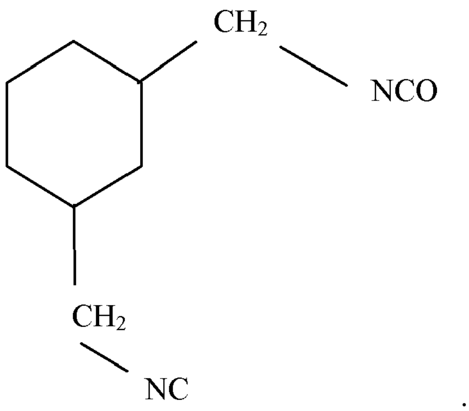

Comparative Example 3

A battery was fabricated in the same manner as described in Example above, except that the addition agent to be added to the non-aqueous electrolyte was an addition agent represented by the following chemical formula (5) (the amount of the addition agent was 1 mass % with respect to the mixed solvent).

The battery fabricated in this manner is hereinafter referred to as Comparative Battery Z3.

Chemical Formula (5)

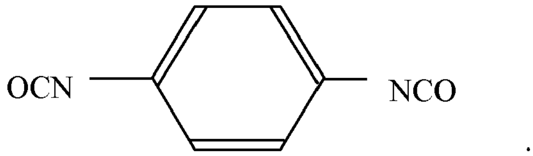

Comparative Example 4

A battery was fabricated in the same manner as described in Example above, except that no addition agent was added to the non-aqueous electrolyte.

The battery fabricated in this manner is hereinafter referred to as Comparative Battery Z4.

Experiment

Each of Battery A of the invention and Comparative Batteries Z1 to Z4 was charged and discharged in the following order: the first charge and discharge, the second charge and discharge, and the third charge, under the following conditions, to determine the open circuit voltage (the open circuit voltage before high-temperature storage) for each battery. Thereafter, each battery was stored at a high temperature under the following conditions, and then, the open circuit voltage (the open circuit voltage after high-temperature storage) was measured for each battery. Then, the voltage drop obtained by the following equation (1) was determined for each battery. The results are shown in Table 1 below. For the measurements of the open circuit voltages, a 3560 AC m-ohm HiTESTER made by Hioki E. E. Corp. was used.

*Conditions for the First Charge and Discharge*

- Charge conditions

Each of the batteries was charged at a constant current of 0.2 It (190 mA) until the battery voltage reached a predetermined voltage (4.20 V), and thereafter charged at a predetermined voltage until the current value reached 0.05 It (48 mA).

- Discharge conditions

Each of the batteries was discharged at a constant current of 0.2 It (190 mA) until the battery voltage reached 2.75 V.

*Conditions for the Second Charge and Discharge*

- Charge conditions

Each of the batteries was charged at a constant current of 1.0 It (950 mA) until the battery voltage reached a predetermined voltage (4.20 V), and thereafter charged at a predetermined voltage until the current value reached 0.05 It (48 mA).

- Discharge conditions

Each of the batteries was discharged at a constant current of 1.0 It (950 mA) until the battery voltage reached 2.75 V.

*Conditions for the Third Charge*

Each of the batteries was charged under the same charge conditions as in the second charge.

All the just-described charge and discharge operations were carried out at 25°C.

*Storage Conditions*

Each battery was stored in a thermostatic chamber at 60°C for 20 days.

*Determination of Voltage Drop*

$$\text{Voltage drop} = \text{Open circuit voltage before the high-temperature storage} - \text{Open circuit voltage after the high-temperature storage} \quad \ldots (1)$$

TABLE 1

| Battery | Type of additive and the amount added | | | | Voltage drop (V) |
|---|---|---|---|---|---|
| | Chemical formula (2) | Chemical formula (3) | Chemical formula (4) | Chemical formula (5) | |
| A | 1 mass % | - | - | - | 0.10 |
| Z1 | - | 1 mass % | - | - | 0.16 |
| Z2 | - | - | 1 mass % | - | 0.18 |
| Z3 | - | - | - | 1 mass % | 0.15 |
| Z4 | - | - | - | - | 0.15 |

As clearly shown in Table 1, the results were as follows. Comparative Battery Z1, containing an addition agent having isocyanato groups but having a linear hydrocarbon (i.e., not having an aromatic hydrocarbon), showed a voltage drop of 0.16 V. Comparative Battery Z2, containing an addition agent in which the isocyanato groups are bonded to a cyclic saturated hydrocarbon via methyl groups (i.e., which has a cyclic saturated hydrocarbon in place of the aromatic hydrocarbon), showed a voltage drop of 0.18 V. Comparative Battery Z3, containing an addition agent in which the isocyanato groups are bonded directly to the aromatic hydrocarbon (i.e., in which no linear saturated hydrocarbon exists between the isocyanato groups and the aromatic hydrocarbon), showed a voltage drop of 0.15 V. Comparative Battery Z4, not containing such an additive agent, showed a voltage drop of 0.15 V.

In contrast, Battery A of the invention, containing the compound having a structural unit in which a plurality of isocyanate structures are provided and also the isocyanato groups are not directly bonded to the aromatic hydrocarbon (i.e., the isocyanato groups are bonded to the aromatic hydrocarbon via the linear saturated hydrocarbon), exhibited a voltage drop of 0.10 V, which was less than those of Comparative Batteries Z1 to Z4.

From the foregoing, it will be appreciated that the high-temperature storage performance is improved by adding a compound that is an aromatic hydrocarbon derivative and that has a structural unit in which the isocyanato groups are not directly bonded to the aromatic hydrocarbon (i.e., the isocyanato groups are bonded to the aromatic hydrocarbon via the linear saturated hydrocarbon). Although it is not clearly shown in the above-described experiment, the present inventors found that the high-temperature storage performance could not be improved sufficiently when the addition agent contained only one isocyanate structure, and that a plurality of isocyanate structures are required in order to improve the high-temperature storage performance sufficiently.

The present invention is suitable for drive power sources for mobile information terminals such as mobile telephones, notebook computers, and PDAs, especially for use in applications that require a high capacity. The invention is also expected to be used for high power applications that require continuous operations under high temperature conditions, such as HEVs and power tools, in which the battery operates under severe operating environments.

While detailed embodiments have been used to illustrate the present invention, to those skilled in the art, however, it will be apparent from the foregoing disclosure that various changes and modifications can be made therein without departing from the spirit and scope of the invention. Furthermore, the foregoing description of the embodiments according to the present invention is provided for illustration only, and is not intended to limit the invention.

## Claims

1. A non-aqueous electrolyte secondary battery comprising: a positive electrode; a negative electrode containing a negative electrode active material; a separator interposed between the positive and negative electrodes; and a non-aqueous electrolyte containing a non-aqueous solvent and a solute dissolved in the solvent, wherein the non-aqueous electrolyte contains a compound represented by the following chemical formula (1):

Chemical formula (1)

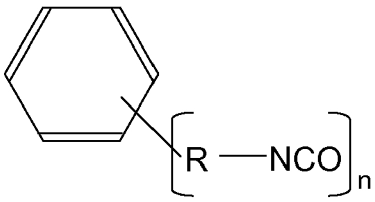

,

wherein n is an integer of from 2 to 6, each R represents a linear saturated hydrocarbon that may be an unsubstituted group or may have a substituted group, and the Rs may be the same or different groups.

2. The non-aqueous electrolyte secondary battery according to claim 1, wherein the negative electrode active material contains silicon.

3. The non-aqueous electrolyte secondary battery according to claim 1 or 2, wherein the R in the chemical formula (1) is a linear saturated hydrocarbon having a carbon number of from 1 to 4.

4. The non-aqueous electrolyte secondary battery according to claim 3, wherein the R in the chemical formula (1) is a methylene group.

5. The non-aqueous electrolyte secondary battery according to any one of claims 1 through 4, wherein n in the chemical formula (1) is 2.

6. The non-aqueous electrolyte secondary battery according to claim 5, wherein the two R-NCOs in the chemical formula (1) are in the meta position.

7. The non-aqueous electrolyte secondary battery according to any one of claims 1 through 6, wherein the non-aqueous solvent contains a cyclic carbonate ester and a chain carbonate ester, and at least one of the carbonate esters contains fluorine.

FIG. 1

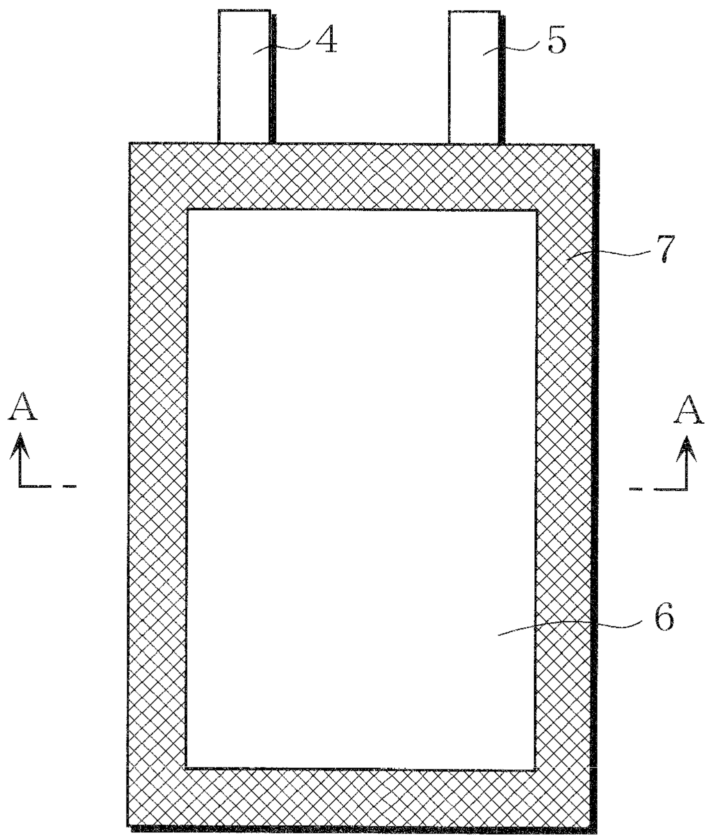

FIG. 2

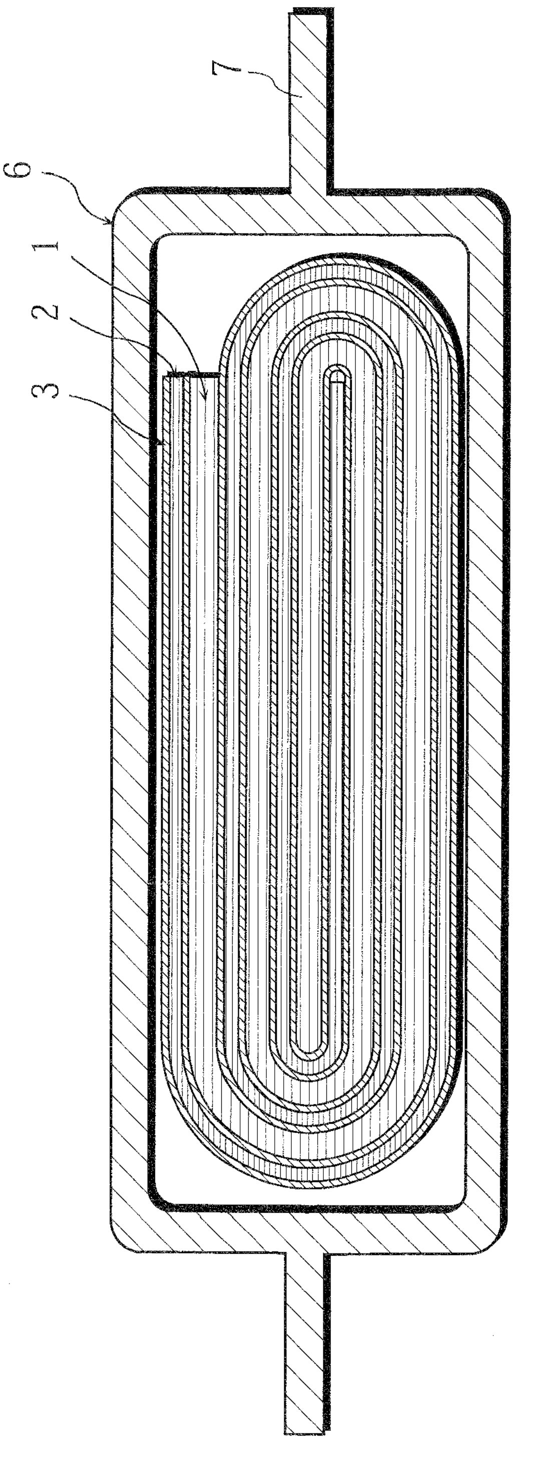

Europäisches Patentamt
European Patent Office
Office européen des brevets

# EUROPEAN SEARCH REPORT

Application Number
EP 11 15 4818

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| E<br><br>L | EP 2 320 499 A1 (SANYO ELECTRIC CO [JP]) 11 May 2011 (2011-05-11)<br>* L so quoted as doubt on the priority *<br>* page 3, paragraph 15 - page 4, paragraph 16 *<br>* page 4, paragraph 24 - page 5 *<br>* page 5, paragraph 27 *<br>* page 6, paragraph 38 - page 7, paragraph 42 *<br>* page 12, line 86 *<br>* page 12, paragraph 90 - page 13, paragraph 91 *<br>* page 14, paragraph 99 *<br>----- | 1-7<br><br>1-7 | INV.<br>H01M10/052<br>H01M10/0567 |
| A | US 6 905 762 B1 (JOW T RICHARD [US] ET AL) 14 June 2005 (2005-06-14)<br>* the whole document *<br>----- | 1-7 | |
| A | JP 2006 164759 A (TOMIYAMA PURE CHEMICAL IND LTD) 22 June 2006 (2006-06-22)<br>* abstract *<br>----- | 1-7 | TECHNICAL FIELDS SEARCHED (IPC) |
| A | JP 2007 242411 A (SONY CORP) 20 September 2007 (2007-09-20)<br>* abstract *<br>----- | 1-7 | H01M |
| A,P | EP 2 190 054 A1 (MITSUBISHI CHEM CORP [JP]) 26 May 2010 (2010-05-26)<br>* page 3, paragraph 18 - page 4, paragraph 21 *<br>* page 6, paragraph 27 - paragraph 30 *<br>* page 22, paragraph 138 - paragraph 139 *<br>* table 1 *<br>----- | 1-7 | |

1

The present search report has been drawn up for all claims

| Place of search | Date of completion of the search | Examiner |
|---|---|---|
| The Hague | 24 May 2011 | Gamez, Agnès |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 11 15 4818

This annex lists the patent family members relating to the patent documents cited in the above-mentioned European search report.
The members are as contained in the European Patent Office EDP file on
The European Patent Office is in no way liable for these particulars which are merely given for the purpose of information.

24-05-2011

| Patent document cited in search report | Publication date | Patent family member(s) | Publication date |
|---|---|---|---|
| EP 2320499 A1 | 11-05-2011 | WO 2010021236 A1 | 25-02-2010 |
| US 6905762 B1 | 14-06-2005 | NONE | |
| JP 2006164759 A | 22-06-2006 | NONE | |
| JP 2007242411 A | 20-09-2007 | NONE | |
| EP 2190054 A1 | 26-05-2010 | WO 2009035054 A1 | 19-03-2009 |
| | | JP 2009087934 A | 23-04-2009 |
| | | KR 20100057612 A | 31-05-2010 |
| | | US 2011091768 A1 | 21-04-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2007242411 A